Europäisches Patentamt

(19) European Patent Office

Office européen des brevets

(11) **EP 1 224 871 A2**

(12) **DEMANDE DE BREVET EUROPEEN**

(43) Date de publication:
**24.07.2002 Bulletin 2002/30**

(51) Int Cl.⁷: **A23L 1/337**, A01K 61/00

(21) Numéro de dépôt: **01402999.5**

(22) Date de dépôt: **21.11.1994**

(84) Etats contractants désignés:
**AT BE CH DE DK ES FR GB GR IE IT LI LU MC NL PT SE**

(30) Priorité: **22.11.1993  FR 9314141**

(62) Numéro(s) de document de la (des) demande(s) initiale(s) en application de l'article 76 CBE:
**94420324.9 / 0 655 250**

(71) Demandeurs:
  • **Texinfine S.A.**
    **69400 Lyon (FR)**
  • **PATRINOVE**
    **69006 Lyon (FR)**

(72) Inventeur: **Gutierrez, Gilles**
    **69006 Lyon (FR)**

(74) Mandataire: **Burtin, Jean-François**
    **Cabinet GEFIB,**
    **82, rue Baudin**
    **92300 Levallois-Perret (FR)**

Remarques:
    Cette demande a été déposée le 29 - 11 - 2001 comme demande divisionnaire de la demande mentionnée sous le code INID 62.

(54) **Compositions à bases d'algues Padina ou de leurs extraits et leurs applications alimentaires ou pour la culture de mollusques ou d'arthropodes**

(57)    La présente invention se rapporte au domaine des compositions à base d'algues Padina ou de leurs extraits et leurs applications alimentaires ou pour la culture de mollusques ou d'arthropodes.

Ces compositions trouvent des applications dans des compositions alimentaires d'apport nutritif en cal-cium, dans des compositions destinées à améliorer la fixation du calcium des cellules nacrifères des mollusques ainsi que dans des compositions destinées à accélérer le processus de formation des perles et des arthropodes.

**EP 1 224 871 A2**

**Description**

La présente invention concerne l'utilisation d'algues du genre Padina ou d'extraits de ces algues pour la réalisation des compositions destinées à améliorer la fixation du calcium par les tissus biologiques, leurs applications dans le domaine alimentaire ainsi que dans la culture des mollusques perliers et nacrifères et des arthropodes.

On connaît de nombreuses algues dont certaines sont classées parmi les algues calcifiantes en raison de leurs thalles fortement calcifiés ou dits « encroûtants ».

C'est ainsi que l'on peut citer Sphaerococcus coronopifolius (algue rouge, Rhodophycée), Liagora Viscida (algue rouge) Halimeda tuna (algue verte, Chlorophycée).

L'inventeur a constaté que parmi les nombreuses algues calcifiantes du type phéophycées, et notamment les dictyotales, certaines espèces du genre Padina, dont la plus commune est dénommée Padina pavonica (pavonia ou padine), présentaient des propriétés particulières et originales, que l'on ne retrouvait pas chez les autres algues de ce type. C'est ainsi qu'à côté de la Padina pavonica, on trouve aux USA une autre variété de cette espèce communément dénommée Zonaria, et dans d'autres régions du globe, on trouve des algues calcifiantes du même type telles la Padina vickersiae et la Tonia atomaria, etc.

Dans ce qui suit, et dans un but de simplification, les algues de ce type seront simplement dénommées du type « Padina pavonica », ce terme correspondant au genre Padina.

Certaines de ces algues ont déjà trouvé une utilisation dans le domaine alimentaire. C'est ainsi que JP-A-62 087 071 (Database WPI DW 8721) décrit leur utilisation, en même temps que des extraits de légumes et de fruits dans les régimes amaigrissants.

JP-A-62 099 331 (Database WPI DW 8724) décrit des préparations alimentaires à base de feuilles de carottes renfermant éventuellement une ou plusieurs algues et notamment la Padina, ces préparations étant utilisées pour prévenir le cancer.

L'inventeur a déterminé, de façon surprenante, que ces algues présentaient des propriétés leur permettant d'améliorer la fixation du calcium dans les tissus biologiques, notamment chez les mammifères, les mollusques et les arthropodes.

Il a pu par ailleurs constater que cette activité n'était pas inhibée lors de la digestion de l'algue ou de ses extraits par des enzymes protéolytiques.

Les algues du type Padina pavonica trouvent un emploi intéressant comme complément alimentaire d'apport en calcium.

Enfin elles peuvent être utilisées pour améliorer la fixation du calcium des cellules nacrifères des mollusques et pour accroître la vitesse de formation de l'aragonite et de la calcite lors de la culture des perles et la production de la nacre.

La présente invention sera mieux comprise et ses avantages ressortiront bien de la description qui suit, qui l'illustre sans nullement la limiter.

Le processus d'obtention va tout d'abord être décrit en détail.

Les algues Padina pavonica sont ramassées à une profondeur variant de 30 cm à 20 m au-dessous du niveau de la mer. Elles sont ensuite lavées à l'eau de mer et débarrassées des différents parasites ou polluants (autres algues, petits animaux, débris de coquillages...) et séchées sur des claies bien aérées. La masse sèche ainsi obtenue correspond à peu près à 20 % de la masse d'algues humides.

Bien sèche, l'algue est facilement broyable, soit manuellement grâce un mortier, soit à l'aide d'appareils (broyeur). La poudre présente une teneur en calcium d'environ 10 % selon les sites, la profondeur et l'âge de la plante.

100 mg de cette poudre ont été mis en suspension dans 5 ml d'éthanol pur. L'analyse de la solution alcoolique, appelée dans ce qui suit « extrait de Padina pavonica » (ou EPP) n'a plus révélé la présence de calcium.

Cet EPP renferme 10 mg de matière extractive pour 100 mg d'algues. L'EPP a été testé *in vitro* sur des cellules ostéoblastiques (lignée G 292, ECACC) selon les techniques classiques de culture cellulaire que l'on va rappeler brièvement :

Les cellules sont ensemencées dans des flasquettes de 75 cm$^2$ à une densité de 2,5 x 10$^4$ cellules/cm$^2$.

Le milieu de culture utilisé est un milieu Mc Koy 5 sans sérum de veau foetal. Après confluence, les cellules sont trypsinées, comptées puis ensemencées dans des plaques à 24 puits, à une densité de 5.10$^5$ cellules par puits. On rajoute, dans chaque puits, 0,5 ml de milieu de culture contenant les différentes quantités d'Epp que l'on désire tester.

Les témoins servant de référence sont obtenus par adjonction, dans leur milieu de culture, d'une quantité d'alcool correspondant à celle qui est introduite par l'Epp lors des essais.

Les résultats, rassemblés dans le tableau 1 suivant, sont exprimés en ng/ml pour 10$^6$ cellules après 48 heures de traitement. Le dosage du calcium a été réalisé à l'aide d'un spectrophotomètre atomique d'adsorption sur la totalité du calcium fixé intra et extra cellulaire.

TABLEAU 1

| TEMOIN | | |
|---|---|---|
| Alcool | 5 µl | 25 µl |
| Teneur en calcium | 120 ng | 105 ng |
| DOSES | 50 µg | 250 µg |
| Teneur en calcium | | |
| Epp | 332 ng | 396 ng |
| Extrait aqueux | 107 ng | 97 ng |

Ces résultats montrent que les cellules témoins fixent 120 ng de calcium par million de cellules cultivées en milieu Mc Koy ; après traitement par un extrait aqueux de padine, on n'observe aucun changement.

L'utilisation de 50 µg d'Epp (en solution alcoolique) induit une augmentation de la fixation du calcium d'un facteur de 3. Cette augmentation est d'environ 4 si l'on utilise 250 µg d'Epp (en solution alcoolique).

L'algue Padina pavonica ne peut présenter un intérêt dans la fixation du calcium dans l'os que si elle permet un traitement facilement utilisable, ce qui implique la possibilité de prise par voie orale.

L'inventeur a donc comparé l'activité de l'Epp d'une algue traitée par différents enzymes digestifs protéolytiques (enzymes stomacaux, notamment pepsine). Les résultats sont rassemblés dans le tableau II suivant.

TABLEAU II

| TEMOIN Alcool | | |
|---|---|---|
| Concentration | 5 µl | 25 µl |
| Teneur en calcium | 121 ng | 103 ng |
| DOSES | 50 µg | 250 µg |
| Teneur en calcium | | |
| Epp | 235 ng | 307 ng |
| Epp sur une algue traitée par la pepsine | 189 ng | 284 ng |

On constate donc qu'il n'existe pas de différences significatives entre l'activité de l'Epp d'une algue pure et l'Epp d'une algue traitée par un enzyme d'origine stomacale.

La variation observée correspond à la perte du principe actif solubilisé dans l'alcool et dispersé dans la solution aqueuse lors du traitement enzymatique.

Afin de valider pleinement les expériences, l'inventeur a testé différentes algues et notamment d'autres algues dites calcifiantes, telles que la Sphaerococcus coronopifolius (SC), l'Amphiora sp (AS) et l'Halimeda tuna (HT). Les résultats, rassemblés dans le tableau III, ont été obtenus dans les conditions suivantes :

Après traitement (lavage et élimination des différents polluants comme mentionné ci-avant) les algues ont été séchées et 100 mg de poudre sèche ont été traités par 5 ml d'alcool.

TABLEAU III

| TEMOIN Alcool | | |
|---|---|---|
| Concentration | 5 µl | 25 µl |
| Teneur en calcium | 120 ± 4,4 ng | 103 ± 5,2 ng |
| DOSES | 50 µg | 250 µg |
| Teneur en calcium | | |
| SC | 97 ± 1,8 ng | 107 ± 2,2 ng |
| AS | 116 ± 2,3 ng | 110 ± 1,6 ng |
| HT | 107 ± 2,4 ng | 97 ± 5,8 ng |
| Epp | 282 ± 5,4 ng | 313 ± 1,9 ng |

Des essais similaires effectués sur d'autres algues, calcifiantes ou non, comme la Fosliella farinosa, l'Udotea Petiolata, l'Asparagopsis armata, la Dictyota linearis, la Codium vermilara n'ont pas permis de noter de changements notables par rapport aux résultats précédents.

Il a paru par ailleurs important de situer l'activité de la Padina pavonica par rapport à certains produits habituellement cités comme intervenants majeurs dans la fixation du calcium par les ostéoblastes...

Dans ce but, ne connaissant pas l'actif présent dans l'Epp, l'inventeur a travaillé avec des poids secs extrêmement précis.

Le poids sec de l'Epp a été obtenu par évaporation de l'alcool à une température de 70°C pendant 5 heures.

Le produit a ensuite été testé par rapport à différents produits :

- la calcitonine (dont la dose thérapeutique est de l'ordre de $10^{-7}$ M dans le plasma)
- la vitamine D 3.
- l'oestradiol
- l'hormone parathyroïdienne (Hpth).

Les résultats obtenus sont rassemblés dans le tableau IV.

TABLEAU IV

|  | Doses | Teneur en calcium en ng/ml |
|---|---|---|
| Témoin alcool | 10 μl | 121 ± 3 |
| Témoin alcool | 5 μg | 137 ± 11 |
| Calcitonine | 35 μg | 791 ± 24 |
| Oestradiol | 2,7 μg | 569 ± 12 |
| Vitamine D 3 | 3,8 μg | 192 ± 6 |
| Hpth | 100 μg | 469 ± 23 |
| Epp (poids sec) | 5 μg | 363 ± 10 |

Les résultats obtenus permettent de constater que l'activité de l'Epp est environ 3 fois supérieure à celle de la calcitonine, du même ordre de grandeur que celle de l'oestradiol, de deux fois supérieure à celle de la vitamine D 3 et de 20 fois supérieure à celle de l'Hpth.

Pour déterminer les doses d'emploi chez les mammifères et notamment chez l'homme, on a admis que l'algue pouvait être employée pure, et que, par conséquent, le sérum (du sang) pouvait être comparé au milieu de culture avec son adjonction d'Epp.

La dose active serait d'environ 250 μg/ml (2,5 / 10000) d'une solution de 100 mg / 5 ml (2 %).

Le volume sanguin est d'environ 5 litres, composé pour moitié de sérum, soit 2,5 l. ou 2 500 ml pour un adulte d'un poids moyen de 75 kg.

Pour cet adulte, la dose en gramme sera de :

$$2\ 500 \times 2,5 / 10\ 000 \times 2 / 100 = 12,5000 / 1000000 = 12,5 / 1000$$

Soit 12,5 mg par prise ou 0,166 mg / kg.

L'éventuelle toxicité de cette algue a été ensuite examinée en procédant, chez des souris, à un gavage avec des doses 10, 100, 500 et 1000 fois supérieures, soit jusqu'à 166 mg/kg ce qui correspondrait à une dose de 12,450 g chez un homme adulte.

Aucune anomalie n'est apparue et il n'a pas été possible de déterminer la DL 50 (dose létale tuant 50 % des animaux).

Il a été ensuite procédé à un essai thérapeutique dans les conditions suivantes :

6 volontaires sains âgés de 37 à 74 ans ont accepté de prendre des comprimés de composition suivante :

- poudre de Padina pavonica : 50 mg
- silice, type Sippernat : 5 mg
- cellulose microcristalline : q.s.p. un comprimé de 300 mg.

Un bilan osseux a été réalisé en début de traitement ; le traitement a duré 6 mois à raison de 4 comprimés dosés à 50 mg par jour. Deux autres bilans osseux ont été réalisés, au bout de 3 mois et en fin de protocole.

Le calcium osseux a été évalué par absorptiométrie biphotonique (appareil de type Lunar DPX de marque Hologic) sur les vertèbres lombaires L1, L2, L3 et L4 ainsi que sur le poignet gauche. Une radiographie de ces sites a été pratiquée afin d'éliminer les arthroses qui pourraient modifier les résultats par inclusion de la masse osseuse des épiphytes (petites excroissances osseuses dues à la maladie rhumatismale).

L'appareil exprime les résultats en masse de calcium par $cm^2$ de surface cutanée traversée, déduction faite de la surface sous laquelle il n'y a pas d'os.

Sa précision est de l'ordre de 10 mg pour une analyse d'une surface de 15,00 $cm^2$. Les résultats ont montré une augmentation de l'ordre de 1 à 4 % de la masse de calcium en 77 jours.

Une deuxième évaluation clinique a été faite pour déterminer, à court terme, les préventions de la dégradation de la matrice extracellulaire osseuse en dosant l'hydroxyproline urinaire et la pyridinoline.

Cette dégradation de la matrice précède l'apparition des troubles de l'ostéoporose et plus généralement ceux liés à une fuite du calcium au cours de l'âge, sans que celle-ci soit pathologique.

La pyridinoline est un marqueur de la réticulation des fibres collagéniques de la matrice extracellulaire osseuse, l'hydroxyproline est un marqueur de la matrice extracellulaire collagénique, entre autres de la matrice extracellulaire osseuse. On a aussi pu noter une augmentation très significative de ces marqueurs en quelques semaines, traduisant un remaniement de l'os.

Il est bien entendu envisageable d'utiliser des formes galéniques autres que les comprimés, telles que les gélules, les poudres ainsi que les solutions injectables et orales.

On conçoit facilement tout l'intérêt des algues du genre Padina ou de leurs extraits comme ingrédient dans les compositions pharmaceutiques destinées à traiter et à améliorer les maladies des os telles que l'ostéoporose, l'arthrose, les troubles fonctionnels associés au mouvement calcique entre le sang et un tissu, les cicatrisations post opératoires ou traumatiques, les fuites calciques dues au vieillissement, ainsi qu'à prévenir ces maladies chez les sujets sains.

Les algues du genre Padina peuvent trouver un emploi intéressant comme complément alimentaire d'apport en calcium.

Par ailleurs l'inventeur a testé l'activité de l'Epp sur un certain nombre de mollusques, notamment les conchifères bivalves qui synthétisent des nacres ou des perles.

Habituellement, les perles de culture sont obtenues en 3 ans, et la nacre est constituée par un arrangement régulier de calcite sur un lit d'aragonite maintenu par des protéines de structure.

Les essais ont été effectués sur deux espèces de bivalves : Mytilus provincialis (mollusque perlier) et Crasostrea angulata (mollusque nacrifère), dont les cellules ont été dissociées, à partir de prélèvements sur le bord du manteau, à l'aide d'une solution de pronase E puis cultivées dans un milieu adéquat (Lenoir et Mathieu, 1986) à l'abri de la lumière et à une température de 15°C.

Le dosage du calcium a été réalisé à l'aide d'un spectrophotomètre atomique d'absorption sur la totalité du calcium intra et extra cellulaire.

Les résultats, exprimés en µg de calcium pour 1 million de cellules après 72 heures d'incubation, sont rassemblés dans le Tableau V ci-après.

TABLEAU V

|  | Témoin | Epp 100 µg |
|---|---|---|
| Crassostrea | 30 ± 0,40 ng/millions de cellules | 61,48 ± 29 ng/millions de cellules |
| Mytilus | 3,03 ± 1,5 ng/millions de cellules | 7,3 ± 2 ng/millions de cellules |

On remarque une augmentation très significative de la teneur en calcium fixé, ce qui confirme tout l'intérêt de l'Epp dans la culture des conchifères et autres mollusques perliers et nacrifères.

L'activité de l'Epp a été également déterminée sur les arthropodes.

De nombreux animaux présentent, comme certains mollusques, un exosquelette porté par une matrice en chitine. Ces animaux à articulation extérieure sont appelés arthropodes. Parmi eux se trouvent les artemias qui présentent l'intérêt d'être facilement élevables en laboratoire.

Les artemias sont présentés dans le commerce sous forme d'oeufs qu'il suffit de disposer dans l'eau pour provoquer l'éclosion ; celle-ci apparaît au bout de 2 à 4 jours selon les conditions. Après éclosion, les larves sont nourries avec la spiruline additionnée des ingrédients nécessaires à la solubilisation de l'extrait de padine, tels que la bétaïne, les lécithines...

Les résultats sont représentés dans le Tableau VI ci-après :

TABLEAU VI

|  | Taille à 15 jours |
|---|---|
| Témoin | 6 mm |

TABLEAU VI (suite)

|  | Taille à 15 jours |
|---|---|
| Artemia traité par la padine | 12 mm |

Il est bien évident que les résultats constatés se retrouvent chez d'autres espèces d'arthropodes.

**Revendications**

1. Utilisation d'algues du genre Padina ou d'extraits de ces algues pour la réalisation de compositions alimentaires destinées à améliorer la fixation du calcium par les tissus biologiques.

2. Utilisation d'algues du genre Padina ou d'extraits de ces algues selon la revendication 1, comme complément alimentaire d'apport en calcium.

3. Compositions alimentaires destinées à améliorer la fixation du calcium des cellules nacrifères des mollusques, **caractérisées en ce qu'**elles utilisent des algues du genre Padina sur un support ou un véhicule alimentaire approprié.

4. Compositions alimentaires destinées à accélérer le processus de formation des perles, **caractérisées en ce qu'**elles utilisent des algues du genre Padina selon la revendication 1 sur un support ou un véhicule alimentaire approprié.

5. Compositions alimentaires destinées à accélérer le processus de formation des arthropodes, **caractérisées en ce qu'**elles utilisent des algues du genre Padina selon la revendication 1 sur un support ou un véhicule alimentaire approprié.